# EUROPEAN PATENT SPECIFICATION

(11) **EP 2 491 966 B1**
(45) Date of publication and mention of the grant of the patent: **25.12.2013**
(21) Application number: 11183484.2
(22) Date of filing: 30.09.2011
(51) Int. Cl.: A61M 1/10

(54) **Blood pump, in particular a pneumatic ventricular assist device**
Blutpumpe, insbesondere eine pneumatische ventrikuläre Hilfsvorrichtung
Pompe sanguine, en particulier un dispositif d'assistance ventriculaire pneumatique

(30) Priority: 28.02.2011 PL 39405611
(43) Date of publication of application: 29.08.2012
(73) Proprietor: Fundacja Rozwoju Kardiochirurgii Im. Prof. Zbigniewa Religi, 41-800 Zabrze (PL)
(72) Inventor: Kapis, Artur, 41-800 Zabrze (PL); Kustosz, Roman, 41-800 Zabrze (PL); Bujok, Wojciech, 41-707 Ruda Slaska (PL); Jozwik, Krzysztof, 92-434 Lódz (PL); Obidowski, Damian, 95-020 Stróza (PL); Reorowcz, Piotr, 09-500 Zaborów Stary (PL); Klosinski, Przemyslaw, 99-335 Witonia (PL)
(74) Representative: Malcherek, Piotr

(56) References cited:
- PL-Y1- 63 283
- US-A- 4 167 046
- US-A- 4 222 127
- US-A1- 2002 165 426

## Description

The subject of the invention is a blood pump, in particular a pneumatic ventricular assist device.

The known pneumatic ventricular assist devices ( VAD ) are applied to support the hearts of patients with congestive heart failure. The known ventricular assist device, which is in practice a blood pump, comprises a spherical cap with a blood chamber and an air chamber separated by a membrane. The membrane is mounted around the inside perimeter of the spherical cap. The blood chamber of the spherical cap has an inlet channel for blood and an outlet channel for blood, which channels have circular seats for mounting valves regulating the blood flow through the pump. In the case of external ventricular assist devices mechanical, disc heart valves are applied. Connectors used for connecting the pump to cannulas joining the pump to the circulatory system of a patient are fixed to the inlet and outlet channels with first ends thereof. The pneumatic part of the spherical cap is connected to a generator making alternate pneumatic wave which stimulates alternate movement of the membrane located between the pneumatic part of the spherical cap and the blood chamber thereof. The alternate movement of the membrane results in the blood flowing through the device.

In the known VAD systems the diameters of the seats in the inlet channel and the outlet channel are the same or the diameter of the inlet channel is larger than the diameter of the outlet channel. What is more, the connector along the working length thereof except for the connecting section has an inside channel whose cross-section is partially uniform and partially narrowing towards the second end which is used for connecting with the cannula.

The example of a pneumatic ventricular assist device is known from the Polish utility model description no. Ru. 63283(PL63283).

The invention relates to a blood pump as claimed, in particular a pneumatic ventricular assist device comprising a spherical cap with an air chamber and a blood chamber separated by a membrane mounted around the inside perimeter of the spherical cap. The blood chamber of the spherical cap has an inlet channel for blood and an outlet channel for blood which channels have circular seats for mounting valves regulating the blood flow through the pump. Connectors used for connecting the pump to the cannulas joining the pump with the circulatory system of a patient are fastened to the inlet channel and the outlet channel with the first ends thereof. The essence of the invention is that the diameter of the seat for the valve in the inlet channel is smaller than the diameter of the seat for the valve in the outlet channel, whereas the inside channel of each connector along the full working length thereof is uniformly convergent towards the second end of said connector.

In a preferred embodiment the ratio of the diameters of the seat in the inlet channel and the seat on the outlet channel is between 0.75 and 0.92, preferably 0.83.

It is also advisable when the inside surfaces of the channels of the connectors are inclined in relation to the connector axis at an angle smaller than 5°, preferably 4°.

It is also recommendable that the connector on the outside surface of the second end thereof provided for connecting to a cannula has an external stop which allows to precisely determine the distance at which the cannula should be mounted on the second end of the connector.

It is also advisable when the connector is connected to the cannula with a two-part nut which may also be fixed on the connector by means of a screw fastening or a form-fitting coupling, which allows for the nut to be fastened to the cannula already mounted on the second end of the connector and significantly facilitates the assembly of the connection.

It is also advisable when the length of the nut is smaller than the distance between the extreme end of a coupling element of the connector assigned to said nut and the second end of the connector, owing to this following appropriate mounting of the cannula on the second end of the connector and appropriate seating of the nut, a protruding part of the second end of the connector is visible through the transparent cannula. It allows to assess the correctness of the fastening of the nut and also allows to observe the area of the inflow and outflow of blood into the inlet connector and from the outlet connector during the operation of the device.

It is also advantageous when the two parts of the nut are interconnected by means of a form-fitting guide coupling of longitudinal run with reference to the axis of the nut hole.

The blood pump, in particular the pneumatic ventricular heart assist device according to the invention as claimed optimises the parameters of blood flow on the inlet and outlet of the device owing to the appropriate differentiation of diameters of the seats for valves in the inlet channel and the outlet channel, which results in the analogical differentiation of the size of the valves mounted in said seats and owing to the new shape of the inside channel of the device connectors. It allows to reduce the areas of stagnation with the speed of blood flow too low to ensure sufficient blood flush and to reduce the areas where the shear stress caused by turbulent flow have a hemolytic influence on blood. As a result a reduced risk of intravascular coagulation or thrombosis in the device was achieved. Moreover, turbulences in the area of valves were eliminated, which had an additional advantageous effect on the optimisation of the dynamics of the blood flow in the device with reference to the thrombosis risk. The construction of the coupling of the connector with the cannula ensures a safe connection with reference to the risk of uncoupling during operation, while being convenient for mounting and easily, repeatedly separable.

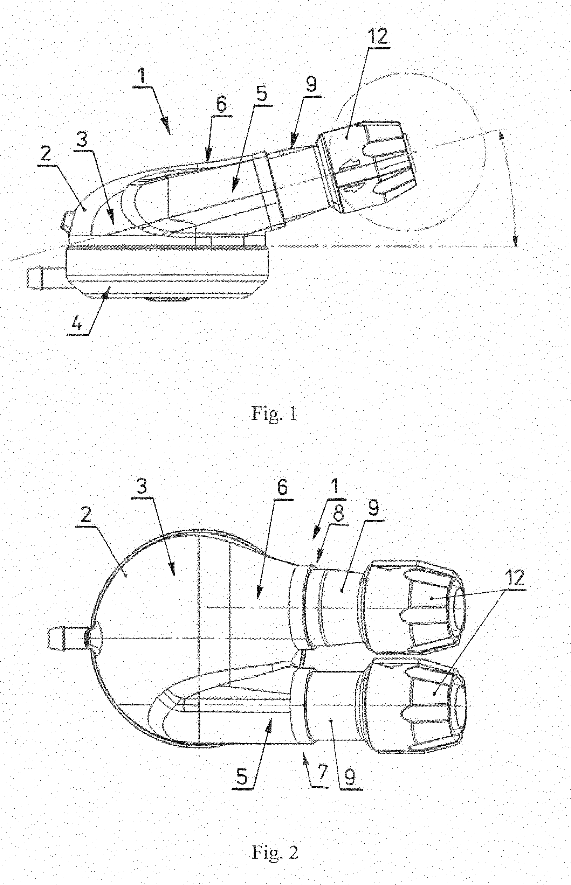

The invention as claimed has been presented in greater detail in the following embodiment and in the enclosed drawing where fig.1 shows a schematic side view of the device, fig.2 - a schematic top view of the device, fig. 3 - schematically presented seats of the inlet channel and of the outlet channel located next to each other, with the ventricular assist device not shown in the figure, fig. 4 - a schematic longitudinal section of the connector, fig. 5 - a close-up of the side view of the nut mounted on the connector with the shown protruding element of the connector, fig. 6 - a cross section of the adjacent parts of the two-part nut, fig. 7 - a longitudinal section of the nut, and fig. 8 - a transverse section of the perspective view of the nut screwed on the second end of the connector.

A blood pump in the form of a pneumatic heart assist device 1 comprises a spherical cap 2 which has an air chamber 4 and a blood chamber 3 separated by a membrane mounted around the inside perimeter of the spherical cap 2. The blood chamber 3 of the spherical cap 2 has an inlet channel 5 for blood and an outlet channel 6 for blood, inside which channels 5, 6 there are circular seats 7, 8 for mounting mechanical valves regulating the flow of blood through the pump. To the inlet channel 5 and the outlet channel 6 are mounted connectors 9 with the first ends thereof, used for coupling the pump with cannulas connecting the pump with the circulatory system of a patient. The diameter D 1 of the seat 7 for the valve in the inlet channel 5 is smaller than the diameter D2 of the seat 8 for the valve in the outlet channel 6.

In the described embodiment the diameter D1 of the seat 7 is 20mm, whereas the diameter D2 of the seat 8 is 24mm. The invention also relates to embodiments where the diameter ratio D1 / D2 is between 0.75 and 0.92. The above difference in the diameters D1 and D2 of the seats 7 and 8 results in different sizes of the mechanical valves mounted in said seats.

The inside channel of each connector 9 along the full working length thereof, except for the connecting section 10, where the connector 9 is mounted on the outlets of the inlet channel 5 and the outlet channel 6, is uniformly convergent towards the second end of said connector 9. In the presented embodiment the angle of the inside surfaces of the connectors 9 with reference to the connector axis is 4°. The invention also relates to embodiments where the angle is smaller than 5°.

On the external surface of the connector 9 at the second end thereof used for coupling with the cannula, there is an external stop 11 which allows to precisely determine the distance at which the cannula should be mounted on the second end of the connector 9.

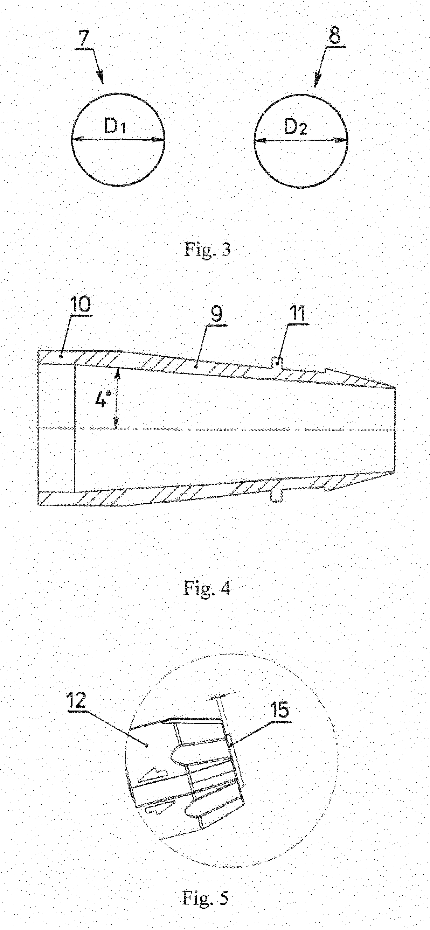

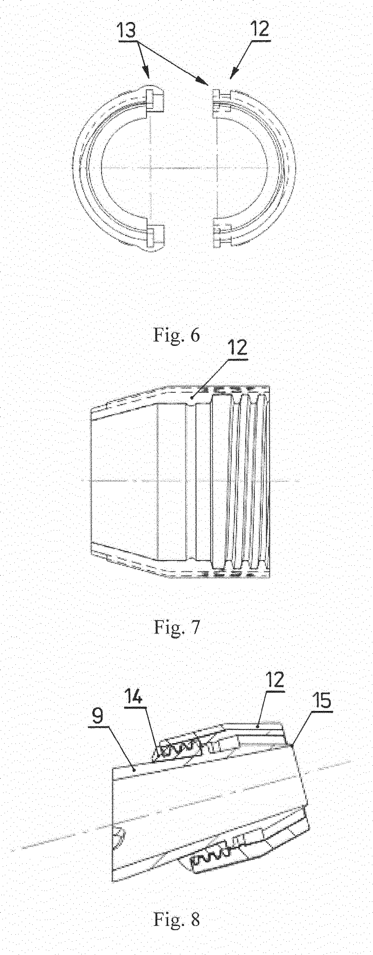

The connector 9 is coupled with the cannula, not shown in the drawing, by means of a two-part nut 12. In the embodiment shown in the drawing the nut 12 consists of two elements interconnected by means of a form-fitting guide coupling 13 with longitudinal run with reference to the nut hole axis. The coupling of the elements of the nut 12 consists in that both halves are mounted on the cannula already mounted on the connector and connects by means of sliding mutually corresponding form-fitting elements of the guide coupling. The nut 12 coupled in this manner is fastened to the connector 9 by means of a screw fastening, which is shown in fig. 8. To achieve this on the external surface of the connector 9 shown in fig. 4 there is a fastened screw element comprising, for example, two halfrings on which the nut 12 is screwed on. Alternatively, instead of the screw fastening any useful form-fitting coupling could be used.

The length of the nut 12 is smaller than the distance between the extreme end 14 of a coupling element of the connector 9, in this case a screw fastening corresponding with the nut and the second end of the connector 9. After appropriate mounting of the cannula on the second end of the connector 9 and screwing on the nut 12 there is a visible protruding element 15 of the second end of the connector 9.

## Claims

1. A blood pump, in particular a pneumatic ventricular assist device (1), comprising a spherical cap (2) with an air chamber (4) and a blood chamber (3) separated by a membrane mounted around the perimeter of the spherical cap, where the blood chamber of the cap has an inlet channel (5) for blood and an outlet channel (6) for blood, where said channels (5,6) have circular seats (7,8) for mounting valves regulating the flow of blood through the pump, to the inlet and outlet channel are fastened connectors (9) with the first ends thereof used for joining the pump to cannulas connecting the pump to the circulatory system of a patient, **characterised in that** the diameter ( D1 ) of the seat ( 7 ) for the valve in the inlet channel ( 5 ) is smaller than the diameter ( D2 ) of the seat ( 8 ) for the valve in the outlet channel ( 6 ), whereas the inside channel of each connector ( 9 ) along the full working length thereof is uniformly convergent towards the second end of said connector ( 9 ).

2. The pump according to claim 1, **characterised in that** the diameter ratio ( D 1 / D2 ) of the seat ( 7 ) of the inlet channel ( 5 ) and of the seat ( 8 ) of the outlet channel ( 6 ) is between 0.75 and 0.92, preferably it is 0.83.

3. The pump according to claim 1, **characterised in that** the internal surfaces of the channels of the connectors ( 9 ) are inclined with reference to the axis of the connector ( 9 ) at an angle smaller than 5°, preferably 4°.

4. The pump according to claim 1 or 2 or 3 **characterised in that** the connector ( 9 ) has an external stop ( 11 ) on the external surface of the second end thereof used for connecting with the cannula.

5. The pump according to one of the above claims **characterised in that** the connector ( 9 ) is fastened to the cannula by means of a two-part nut ( 12 ).

6. The pump according to claim 5 **characterised in that** the nut (12 ) is mounted on the connector ( 9 ) by means of a screw fastening.

7. The pump according to claim 5 **characterised in that** the nut ( 12 ) is mounted on the connector ( 9 ) by means of form-fitting coupling.

8. The pump according to claim 5 or 6 or 7 **characterised in that** the length of the nut ( 12 ) is smaller than the distance between the extreme end ( 14 ) of the connecting element of the connector ( 9 ) for said nut ( 12 ) and the second end of the connector ( 9 ).

9. The pump according to one of the claims 5 to 8 **characterised in that** the two elements of the nut ( 12 ) are interconnected by means of a form-fitting guide coupling ( 13 ) with longitudinal run with reference to the axis of the nut hole ( 12 ).

## Patentansprüche

1. Blutpumpe, insbesondere die pneumatische Herz-Hilfspumpe /1/, ausgerüstet mit der Schale /2/, die die Luftkammer /4/ und die Blutkammer /3/ aufweist, welche mittels am Schalenumfang befestigten Membran voneinander getrennt sind, wobei die Blutkammer /3/ der Schale /2/ mit dem Blut-Einlasskanal /5/ und dem Blut-Auslasskanal /6/ ausgerüstet ist, und die Blut-Kanäle /5, 6/ runde Aufnahmebüchsen /7, 8/ für Aufnahme der Drosselklappen zur Regelung des Blutdurchflusses durch die Blutpumpe aufweisen, und die Anschlusselemente /9/ hingegen, vorgesehen für Anschluss der Blutpumpe an die Kanülen, die die Blutpumpe mit dem Blutkreislauf des Patienten verbinden, sind mit ihren ersten Enden an die Blut-Einlass- und Blut-Auslasskanäle befestigt, **gekennzeichnet dadurch, dass** der Durchmesser /D1/ der Aufnahmebüchse /7/ für die Drosselklappe im Blut-Einlasskanal /5/ kleiner als der Durchmesser /D2/ der Aufnahmebüchse /8/ für die Drosselklappe im Blut-Auslasskanal /6/ ist, jedoch der innere Kanal jedes Anschlusselements /9/ verjüngt sich gleichmäßig seiner ganzen Arbeitslänge entlang zu dem anderen Ende des gegebenen Anschlusselements /9/ hin.

2. Blutpumpe nach Anspruch 1 **gekennzeichnet dadurch, dass** das Verhältnis der Durchmesser /D1:D2/ der Aufnahmebüchse /7/ des Blut-Einlasskanals /5/ und der Aufnahmebüchse /8/ des Blut-Auslasskanals /6/ im Bereich von 0,75 bis 0,92 beinhaltet ist, und vorzugsweise 0,83 beträgt.

3. Blutpumpe nach Anspruch 1 **gekennzeichnet dadurch, dass** die inneren Oberflächen der Kanäle der Anschlusselemente /9/ zur Achse des Anschlusselements /9/ geneigt sind, und der Neigungswinkel weniger als 5°, und vorzugsweise 4° beträgt.

4. Blutpumpe nach Anspruch 1 oder 2 oder 3 **gekennzeichnet dadurch, dass** das Anschlusselement /9/ mit dem Begrenzer /11/ ausgerüstet ist, der an der Außenseite seines zweiten, zur Verbindung mit der Kanüle bestimmten Ende, lokalisiert ist.

5. Blutpumpe nach einem der obigen Ansprüche **gekennzeichnet dadurch, dass** das Anschlusselement /9/ mit einer Kanüle mittels einer aus zwei Elementen bestehenden Schraubenmutter /12/ verbunden ist.

6. Blutpumpe nach Anspruch 5 **gekennzeichnet dadurch, dass** die Schraubenmutter /12/ mittels Schraubenverbindung an dem Anschlusselement /9/ befestigt ist.

7. Blutpumpe nach Anspruch 5 **gekennzeichnet dadurch, dass** die Schraubenmutter /12/ an dem Anschlusselement /9/ über Formschluss befestigt ist.

8. Blutpumpe nach Anspruch 5 oder 6 oder 7 **gekennzeichnet dadurch, dass** die Länge der Schraubenmutter /12/ kleiner als der Abstand zwischen dem äußersten Endpunkt /14/ des Verbindungteils des Anschlusselements /9/ für diese Schraubenmutter /12/ und dem zweiten Ende des Anschlusselements /9/ ist.

9. Blutpumpe nach einem der Ansprüche von 5 bis 8 **gekennzeichnet dadurch, dass** die beiden Elemente der Schraubenmutter /12/ mittels Führungsformschluss /13/, welcher den Längsverlauf zur Achse der Öffnung der Schraubenmutter /12/ aufweist, miteinander verbunden sind.

## Revendications

1. Pompe à sang, en particulier la chambre d'assistance cardiaque, à commande pneumatique (1), munie d'un boîtier (2), dans lequel on peut distinguer une chambre à air (4) et une chambre à sang (3), séparées par une membrane fixée autour de la circonférence interne du boîtier, où la chambre à sang (3) du boîtier (2) a un canal d'entrée (5) pour le sang et un canal de sortie (6) pour le sang, et dans ces canaux (5, 6) on peut distinguer des logements ronds (7, 8) pour y mettre des valvules dans l'objectif de régler le flux de sang à travers la pompe, et aux canaux d'entrée et de sortie sont fixés avec leurs premières extrémités des connecteurs (9) pour relier la pompe à des canules qui raccordent la pompe au système sanguin du patient, **caractérisée en ce que** le diamètre (D1) du logement (7) pour la valvule dans le canal d'entrée (5) est inférieur au diamètre (D2) du logement (8) pour la valvule dans le canal de sortie (6), tandis que le canal intérieur de chaque connecteur (9) sur toute sa longueur de travail est uniformément convergent dans la direction de la seconde extrémité du connecteur (9).

2. Pompe selon la revendication 1, **caractérisée en ce que** le rapport de diamètres (D1/D2) du logement (7) du canal d'entrée (5) et du logement (8) du canal de sortie (6) est dans la gamme de 0,75 à 0,92, de préférence 0,83.

3. Pompe selon la revendication 1, **caractérisée en ce que** les surfaces internes des canaux des connecteurs (9) sont inclinées par rapport à l'axe du connecteur (9) sous un angle inférieur à 5 °, de préférence de 4 °.

4. Pompe selon les revendications 1 ou 2 ou 3, **caractérisée en ce que** le connecteur (9) présente sur la surface extérieure de sa seconde extrémité servant à se connecter à la canule un limiteur externe (11).

5. Pompe selon l'une des revendications mentionnées ci-haut, **caractérisé en ce que** le connecteur (9) est relié à la canule au moyen d'un écrou bi-pièce (12).

6. Pompe de la revendication 5, **caractérisée en ce que** l'écrou (12) est monté sur le connecteur (9) au moyen d'un assemblage par vis.

7. Pompe selon la revendication 5, **caractérisée en ce que** l'écrou (12) est monté sur le connecteur (9) au moyen d'un assemblage par forme.

8. Pompe selon la revendication 5 ou 6 ou 7, **caractérisée en ce que** la longueur de l'écrou (12) est inférieure à la distance entre l'extrémité (14) de l'élément d'assemblage du connecteur (9) pour cet écrou (12) et la seconde extrémité do connecteur (9).

9. Pompe selon l'une des revendications 5 à 8, **caractérisée en ce que** les deux éléments de l'écrou (12) interconnectés par un assemblage par forme à glissière (13) ayant un parcours longitudinal par rapport à l'axe de l'orifice de l'écrou (12).
